# EUROPÄISCHE PATENTSCHRIFT

(11) **EP 2 632 359 B1**
(45) Veröffentlichungstag und Bekanntmachung des Hinweises auf die Patenterteilung: **03.07.2019**
(21) Anmeldenummer: 11773008.5
(22) Anmeldetag: 24.10.2011
(51) Int. Cl.: A61B 18/00, A61B 18/12, H05H 1/24, A61B 18/04, A61B 18/14

(54) **BLUTSTILLUNGSINSTRUMENT**
HEMOSTASIS INSTRUMENT
INSTRUMENT HÉMOSTATIQUE

(30) Priorität: 07.12.2010 DE 102010061059; 07.12.2010 DE 102010061058; 26.10.2010 DE 102010060165; 26.10.2010 DE 102010060163
(43) Veröffentlichungstag der Anmeldung: 04.09.2013
(73) Patentinhaber: Erbe Elektromedizin GmbH, 72072 Tübingen (DE)
(72) Erfinder: NEUGEBAUER, Alexander, 72116 Mössingen (DE); FISCHER, Klaus, 72202 Nagold (DE); ENDERLE, Markus D., 72070 Tübingen (DE)
(74) Vertreter: Bohnenberger, Johannes
(86) Internationale Anmeldenummer: PCT/EP2011/068542
(87) Internationale Veröffentlichungsnummer: WO 2012/055816

(56) Entgegenhaltungen:
- DE-A1- 3 642 077
- DE-A1- 19 537 897
- DE-A1-102005 021 304
- DE-A1-102009 002 278
- DE-A1-102010 015 899
- US-A- 5 449 356
- US-A- 5 707 402
- US-A1- 2005 143 726
- US-A1- 2006 084 158
- US-B1- 6 391 027

## Beschreibung

Die Erfindung betrifft ein Blutstillungsinstrument gemäß dem Oberbegriff des Anspruchs 1 sowie eine Chirurgieeinrichtung zur Stillung von Blutungen gemäß dem Oberbegriff des Anspruchs 9.

Viele chirurgische Operationen, insbesondere offene, laparoskopische oder endoskopische Operationen erfordern eine aktive Stillung von auftretenden Blutungen. Treten Blutungen auf, so müssen diese gestoppt werden, da sonst der Operations-Situs schnell mit Blut bedeckt ist und eine effektive Fortführung der Operation oft unmöglich ist. Bei schweren Blutungen besteht darüber hinaus eine akute Verblutungsgefahr des Patienten, wenn die Blutung nicht innerhalb kurzer Zeit zum Stillstand gebracht wird. Im Falle schwerer Blutungen werden oft Blutkonserven benötigt, die prinzipiell Mangelware sind und außerdem von Patienten häufig aus religiösen oder anderen Gründen abgelehnt werden. Darüber hinaus besteht bei der Verwendung von Blutkonserven immer ein gewisses Restrisiko einer Infektion. Herkömmliche Verfahren und Vorrichtungen zur Blutstillung bedienen sich mechanischer, elektrochirurgischer oder laserchirurgischer Mittel. Weiterhin besteht die Möglichkeit, Fibrinkleber oder Kollagenauflagen zur Blutstillung zu verwenden. Mechanisch kann eine Naht oder ein Hämoclip zur Blutstillung eingesetzt werden. Die mechanische Blutstillung ist gegenüber der elektrochirurgischen und der laserchirurgischen Blutstillung mit einem erheblich größeren Zeitaufwand und schwierigerer Handhabung verbunden. Ebenfalls können Naht- und Hämoclip-Unverträglichkeiten durch Fremdmaterial, insbesondere durch Fadenmaterial und metallische Werkstoffe auftreten. Hämoclips können wandern, sich öffnen, und im schlimmsten Fall Organe perforieren oder Nachbarstrukturen fälschlicherweise mit "verschließen". Nähte können Adhäsionen bzw. Verwachsungen und Abszesse verursachen. Insbesondere endoskopisch aber auch laparoskopisch sind nicht alle Blutungen aufgrund der Erreichbarkeit der Blutung mit den mechanischen Mitteln möglich. Für diffuse Blutungen sind mechanische Methoden der Blutstillung in der Regel nicht geeignet.

Unter die sogenannte "thermische Blutstillung" fallen alle gängigen Verfahren der Elektrochirurgie und der Laserchirurgie. Zu den elektrochirurgischen Verfahren zählen Verfahren aus dem Bereich der Hochfrequenzchirurgie, der KontaktKlemmen-Koagulation und der kontaktlosen Plasma-Koagulation, wie beispielsweise die Argon-Plasma-Koagulation sowie blutungsarme Hochfrequenzschneidemethoden. Die Blutstillung mittels des Lasers und mittels der Elektrochirurgie zielen auf eine Erwärmung der blutenden Gewebestelle ab, wobei bei der Laserchirurgie eine exogene Erwärmung und bei der Elektrochirurgie eine endogene Erwärmung des Gewebes zur Blutstillung führt. Nachteilig ist hierbei das Auftreten hoher lokaler Temperaturen von bis zu 300°C und ein damit einhergehendes Risiko der thermischen Schädigung von angrenzendem Gewebe. Darüber hinaus heilen die Wunden, welche durch Laser und Elektrochirurgie erzeugt werden im Vergleich zu einer mechanischen Schädigung des Gewebes durch ein Skalpell nur verzögert. In vielen Fällen konnte gezeigt werden, dass auch thermisch induzierte Wunden Verwachsungen von Organen untereinander oder Verwachsungen mit der Bauchwand verursachen können. Besonders problematisch ist dieser Befund bei fast allen Operationen im Bereich der Gynäkologie, aber auch bei anderen operativen Fachdisziplinen. Hiervon sind besonders viele junge Frauen betroffen, da die typischen Operationsindikationen Frauen im gebärfähigen Alter betreffen (Ovarialzysten-Entfernung, Myom-Entfernung, Tubenchirurgie). Wenn Adhäsionen postoperativ auftreten, können als Folge chronische Schmerzzustände, Fertilitätsstörungen und mechanisch bedingte Darmverschlüsse auftreten. Adhäsionen und die sich hieraus ergebende Symptomatik bzw. Folgeoperationen stellen einen erheblichen Kostenfaktor für das Gesundheitswesen dar. Der Laser ist als Blutstillungsverfahren nicht sehr weit verbreitet, da das Verfahren apparativ sehr aufwändig und teuer ist. Im Wesentlichen ergeben sich dieselben Nachteile wie bei der elektrochirurgischen Blutstillung.

Bei der elektrochirurgischen Blutstillung ist wie gesagt immer mit einer thermischen Schädigung des um eine Blutung herum befindlichen Gewebes zu rechnen. Bei thermisch sensiblen Strukturen können hierdurch erhebliche Nebenwirkungen eintreten.

Der wichtigste Nachteil bei allen elektrochirurgischen Verfahren ist das Problem der optimalen Dosierung der elektrischen Energie. Der Chirurg ist auf allgemeine Einstellungsempfehlungen der Hersteller für einen bestimmten chirurgischen Eingriff angewiesen. Hierbei kann die individuelle Situation und der Patient selbst nicht berücksichtigt werden. Hinzu kommt, dass viele Chirurgen eine subjektive Dosierung der elektrischen Energie vornehmen, was oftmals durch evidenzbasierte Erkenntnisse nicht begründbar ist und oftmals zu unnötig großen thermischen Schädigungen bis hin zu schlimmsten Verbrennungen führt. Die Wahl der geeigneten Parameter für eine bestimmte chirurgische Anwendung wie Strom, Stromform, Spannung, Leistung, Pulsation etc., die letztendlich einen elektrochirurgischen Modus charakterisieren, wurde oftmals durch den Systemhersteller empirisch bestimmt und ist häufig weit von einer optimalen, auf die jeweilige Situation abgestimmte und patienten-individuelle Parametrisierung entfernt. Hinzu kommt die enorme Vielzahl an unterschiedlichen Moden, die dem Chirurgen für seine Tätigkeit zur Verfügung stehen. Beispielsweise bietet das Erbe VIO-System zehn verschiedene Moden zur Durchführung einer Blutstillung an. Durch die große Anzahl an Einstellungsmöglichkeiten ist der Chirurg oft damit überfordert, für die zu bewältigende Aufgabe den optimalen Modus herauszusuchen.

Zu der Gruppe der sogenannten "nicht-thermischen Blutstillung" gehören Verfahren, welche einen biochemischen Eingriff an den blutenden Stellen vornehmen. Dazu zählt der Fibrinkleber, der eine Art Gewebeklebstoff ist und der als physiologischer Zweikomponentenklebstoff die Wundränder verklebt, anstelle sie durch klassische Nähte zu verschließen. Ein Vorteil des Fibrinklebers ist eine Schonung besonders empfindlichen Gewebes. Bei den Ausgangsstoffen Fibrin, Thrombin, Faktor XIII und Aprotinin handelt es sich um biologische Substanzen, bei denen eine Infektion mit Krankheitserregern nicht ganz ausgeschlossen werden kann. Die biologischen Komponenten sind sehr teuer. Weiterhin können insbesondere für großflächige Blutungen Kollagenauflagen verwendet werden. Kollagenauflagen sind poröse schwammartige Wundauflagen, die durch Gefriertrocknung aus einer Kollagendispersion gewonnen werden. Sie können Wundsekret aufnehmen und wirken blutstillend. Sie fördern die Bildung und Organisation des körpereigenen Kollagens. Nach Blutkontakt aggregieren die Thrombozyten an den Kollagenfasern und lösen die Gerinnungsreaktion aus. Nachteilig ist, dass diese Auflagen bei akuten Blutungen nicht die erforderliche Effizienz aufweisen und eher für chronische Blutungen geeignet sind.

DE 10 2009 002 278 A1 offenbart beispielsweise eine Vorrichtung, die eine Wundbehandlung durch ein nicht-thermisches Plasma in Kombination mit Ultraschallimpulsen ermöglicht. Dazu ist eine Möglichkeit vorgesehen, Wasser einzuspritzen, um die Einkopplung der Ultraschallwellen in das Gewebe zu verbessern.

US 2006/0084158 A1 befasst sich ebenfalls mit der Wundbehandlung durch kaltes Plasma und weist dazu eine Elektrode auf, die von einem Dielektrikum ummantelt ist.

Bei allen der oben aufgeführten Methoden zur Blutstillung beeinflussen Antikoagulationsmittel, Blutverdünnungsmittel wie Aspirin® oder Marcumar® sowie der Blutdruck die Effizienz einer Blutstillungsmethode erheblich. Bei Patienten, welche eine erblich bedingte Störung in der Blutgerinnung aufweisen, beispielsweise Bluter oder Patienten mit Hämophilie, ist eine Anwendung von Standardmethoden zur Blutstillung oft kontraindiziert.

Auf natürlichem Wege geschieht die Blutstillung nach einer Verletzung durch eine Folge komplexer biochemischer Reaktionen, welche zusammengefasst als Blutgerinnungskaskade bezeichnet werden. Die Blutgerinnungskaskade besteht aus einem intravaskulären und einem extravaskulären System. Im Rahmen der vorliegenden Erfindung ist nur der extravaskuläre Weg entscheidend, so dass dieser im Folgenden kurz dargestellt werden soll: Das extravaskuläre System wird durch eine Verletzung in Gang gesetzt, die den Gewebefaktor (tissue factor) freisetzt. Der aus Gewebefaktor und Faktor VII gebildete Komplex setzt eine Kaskade in Gang, welche die Aktivierung von Thrombin veranlasst. Aktiviertes Thrombin katalysiert die Reaktion von Fibrinogen zu Fibrin (letzter Schritt der Blutgerinnungskaskade). Fibrin liegt zunächst in Form von Fibrinmonomeren vor, welche schließlich zu einem Fibrinnetzwerk vernetzen. Es bildet sich somit ein Fibringerinnsel aus, welches den Blutfluss zum Erliegen bringt.

Durch eine sogenannte "stille elektrische Entladung" ("*dielectric barrier discharge*") kann auf die oben erläuterte Blutgerinnungskaskade Einfluss genommen werden. Hierbei werden die vier Fibrinopeptide des Fibrinogen-Moleküls abgespalten und es entsteht ein Fibrinmonomer. Dieser Abspaltungsprozess, der natürlicherweise von Thrombin katalysiert wird, bildet die Grundvoraussetzung für eine Vernetzung der Fibrinmonomere zu einem Fibringerinnsel. Eine stille elektrische Entladung ist eine elektrische Entladung zwischen zwei Elektroden mit ausreichend hoher Potentialdifferenz, die durch eine isolierende dielektrische Barriere (Isolator, Dielektrikum) getrennt sind. Als Dielektrikum wird eine schwach oder nicht leitende Substanz bezeichnet, deren Ladungsträger im Allgemeinen nicht frei beweglich sind. Ein Dielektrikum kann ein Gas, eine Flüssigkeit oder ein Feststoff sein. Isolatoren werden dann Dielektrika genannt, wenn diese mit magnetischen oder elektrischen Feldern beaufschlagt werden. Im Gegensatz zu einer Funkenentladung verhindert das Dielektrikum bei der stillen elektrischen Entladung die Entwicklung eines Funkens oder Bogens und die Entladung findet entweder in Form einer sogenannten Mikroentladung (FSD = Filamentary Silent Discharge) oder als homogene Entladung (GSD = Glow Silent Discharge) statt. Diese Entladungsformen sind sehr kurz und stromschwach und deshalb relativ lautlos. Da bei der stillen Entladung meist nur Elektronen transferiert werden und die sehr energiereichen Elektronen beim Zusammenstoß mit Gasatomen aufgrund des Masseunterschieds nur sehr wenig Energie auf die entstehenden Ionen übertragen können, ist die Gastemperatur relativ niedrig (kaltes Plasma). Die Abstände zwischen den Elektrodenplatten betragen bei stillen elektrischen Entladungen üblicherweise 0,1 Millimeter bis mehrere Zentimeter. Der Abstand der aktiven Elektrode zum Gewebe beträgt bei der chirurgischen Einrichtung 0,1 mm bis 1 cm. Für die stille elektrische Entladung ist in der Regel eine hohe Wechselspannung im Radiofrequenz- oder Mikrowellenfrequenzbereich erforderlich, die üblicherweise im Bereich von 20 kV liegt. Die Erzeugung einer derart hohen Spannung erfordert besondere Generatoren mit besonders dicht gewickelten Windungen. Diese Geräte sind teuer und werden in elektrochirurgischen Systemen nicht verwendet. Herkömmliche elektrochirurgische Systeme liefern im Übrigen Spannungen von maximal 6 kV.

Eine Vorrichtung zur stillen elektrischen Entladung ist beispielsweise aus der WO 2006/116252 A2 bekannt. Dort wird eine stille Entladung bei relativ hohen Spannungen im Bereich von 20 kV durchgeführt. Eine entsprechende Vorrichtung ist auch aus der WO 2010/009103 A2 bekannt. Dort wird eine Vorrichtung zur Erzeugung einer stillen elektrischen Entladung zur Behandlung von Blutungen der Schleimhaut im Gastrointestinaltrakt beansprucht. Eine tragbare Vorrichtung zur Erzeugung eines Plasmas mit relativ niedriger Temperatur wird in der US 2009/0206062 A1 beansprucht. Die Vorrichtung dient zur Sterilisation und zur Blut-Koagulation. Es handelt sich folglich um eine Blutstillung durch thermische Gewebe-Koagulation ähnlich der Argon-Plasma-Koagulation.

Eine Aufgabe der vorliegenden Erfindung ist es daher, das Auftreten von Blutungen, die durch ein chirurgisches Trauma ausgelöst sein können, durch ein Blutstillungsinstrument so minimal invasiv wie möglich bei gleichzeitig größtmöglicher Effektivität und Patientensicherheit zu stillen.

Zur Lösung dieser Aufgabe wird ein Blutstillungsinstrument zur aktiven Stillung von Blutungen mit den Merkmalen des Anspruchs 1 vorgeschlagen. Es kommt insbesondere nach einer offenen, laparoskopischen oder endoskopischen Operation an einem Patienten zum Einsatz und weist mehrere Blutstillungskomponenten auf, wobei wenigstens die folgenden Blutstillungskomponenten bei dem Blutstillungsinstrument vorgesehen sind:
- Eine thermische Blutstillungseinrichtung zur Gewebekoagulation zum Erzeugen einer Temperatur oberhalb der Koagualationstemperatur von biologischem Gewebe mittels einer Koagulationselektrode;
- eine biochemische Blutstillungseinrichtung zur Erzeugung einer stillen elektrischen Entladung mittels wenigstens einer Entladungselektrode und einer Isoliereinrichtung, wobei die Isoliereinrichtung zwischen der Entladungselektrode und dem zu behandelnden Gewebe angeordnet ist;
- eine Zufuhreinrichtung zum Zuführen von die Blutgerinnung beeinflussenden Stoffen an das zu behandelnde Gewebe, und
- eine Zufuhreinrichtung zum Zuführen von Edelgas an das zu behandelnde Gewebe.

Ein wesentlicher Punkt der Erfindung liegt also darin, dass mehrere Blutstillungskomponenten in einem einzigen Instrument vereint werden, so dass eine individuell auf den Patienten abgestimmte Blutungsstillung erfolgen kann. Dadurch kann eine Blutstillung besonders minimalinvasiv erfolgen, d.h. das behandelte Gewebe wird so schonend wie möglich koaguliert. Hierzu verfügt das erfindungsgemäße Blutstillungsinstrument über die Möglichkeit der biochemischen Blutstillung mittels einer stillen elektrischen Entladung, auch als "dielectric barrier discharge" (dielektrische Barrierenentladung) bekannt, sowie über eine thermische Blutstillung durch exogene oder endogene Erwärmung des biologischen Gewebes. Weiterhin erlaubt das Blutstillungsinstrument eine Zuführung von Edelgas sowie von die Blutgerinnung beeinflussenden Stoffen an das zu behandelnde Gewebe, wie beispielsweise die Zuführung von Fibrinogen, Thrombin, Aprotinin, Gerinnungsfaktoren oder anderen Stoffen, welche die Blutgerinnung beeinflussen und damit die Blutstillung beschleunigen können. Auf diese Weise kann mit ein und demselben Instrument eine schonende Blutstillung durchgeführt werden und zwar in Abhängigkeit vom Zustand des individuellen Patienten. Je nach Art der zu stillenden Blutung kann folglich entweder die thermische Blutstillung und/oder die biochemische Blutstillung zum Einsatz kommen und zusätzlich die Blutgerinnung beeinflussende Stoffen an das zu behandelnde Gewebe geleitet werden. Die Zufuhr eines Edelgases sorgt zum einen dafür, dass eine Plasma-Koagulation durchgeführt werden kann und zum anderen ermöglicht sie die Erzeugung einer stillen elektrischen Entladung unter Verwendung eines herkömmlichen elektrochirurgischen Systems mit einer Spannung von maximal 6 kV. Bei der stillen elektrischen Entladung erhitzt sich das behandelte biologische Gewebe nicht nennenswert, so dass also die Koagulationstemperatur (60°C) von biologischem Gewebe nicht erreicht wird und folglich eine thermische Schädigung von Gewebe durch Koagulation oder gar Karbonisierung oder Vaporisation ausgeschlossen ist. Im Gegensatz zu herkömmlichen Verfahren der Blutstillung wird das Gewebe bei der Blutstillung durch eine stille elektrische Entladung nicht erhitzt, so dass keine Denaturierung der Proteine stattfindet und das Gewebe biochemisch intakt bleibt.
Die vorliegende Erfindung ermöglicht folglich mit einem einzigen Instrument eine optimale Mischung aus thermischer, biochemischer Blutstillung unter Zugabe eventuell erforderlicher gerinnungsfördernder Substanzen.

Das Blutstillungsinstrument gemäß der Erfindung erlaubt wie gesagt die Erzeugung einer stillen elektrischen Entladung in einer Edelgasatmosphäre, z.B. durch die Verwendung von Argon, Helium oder Neon mit deren Hilfe es möglich wird, die benötigten Spannungen zur Erzeugung eines kalten Plasmas (stille elektrische Entladung) unterhalb von 4 kV zu halten. Die stille elektrische Entladung kann kontinuierlich oder pulsierend erfolgen. Das Blutstillungsinstrument bietet beide Möglichkeiten. Die Isoliereinrichtung der vorliegenden Erfindung kann ein Dielektrikum aus Glas, insbesondere aus Quarzglas oder ein anderes geeignetes Glas, Keramik, Glimmer oder ähnliche Materialien verwenden.

Biochemische Blutstillungen haben den Nachteil, dass die Zeitdauer zur Erreichung einer effizienten Blutstillung bei ca. 20 Sekunden liegt. Um die Zeit für die Blutgerinnung weiter verkürzen zu können, bietet die chirurgische Einrichtung gemäß der vorliegenden Erfindung die Möglichkeit einer endogenen oder exogenen Aufheizung des Blutes auf eine Temperatur unterhalb der Koagulationstemperatur von biologischem Gewebe, d.h. auf eine Temperatur unterhalb 60°C. Hierzu kann das Gewebe mittels des erfindungsgemäßen Blutstillungsinstruments durch ein konventionelles Gasplasma schonend vorgewärmt werden, ohne dass es zu einer thermischen Schädigung des Gewebes kommt. Da die Fibrinopeptide durch β-Faltblattstrukturen über Wasserstoffbrückenverbindungen stabilisiert sind, führt die Zuführung der oben beschriebenen thermischen Energie zu einem Aufbrechen dieser Wasserstoffbrückenverbindungen und damit zu einer Destabilisierung der Fibrinopeptide. Auf diese Weise können die Fibrinopeptide durch die Anwendung der stillen elektrischen Entladung leichter abgespalten werden und die Fibrinbildung kann somit schneller einsetzen.

Wie gesagt verfügt das Blutstillungsinstrument gemäß der vorliegenden Erfindung nicht nur über die Möglichkeit einer biochemischen Blutstillung sondern gleichzeitig über die Möglichkeit einer konventionellen thermischen Blutstillung durch Kontakt-Koagulation, Argon-Plasma-Koagulation oder über die Möglichkeit eines blutarmen Hochfrequenzschnitts. Bei der thermischen Blutstillung werden Gewebetemperaturen oberhalb der Koagulationstemperatur erreicht, d.h. Temperaturen oberhalb von 60°C mit den damit einhergehenden allgemein bekannten Gewebeeffekten. Durch die Anwendung von supraphysiologischen Temperaturen oberhalb der Koagulations-temperatur des biologischen Gewebes werden Proteine und Zellstrukturen verändert, was zu Zelltod und nachfolgender Gewebenekrose führen kann. Ein erster Schritt dieser thermisch bedingten Veränderungen ist die Denaturierung von Proteinen sowie RNA, DNA und Zellmembrankomponenten. Wird das Gewebe oberhalb der Koagulationstemperatur weiter erhitzt, werden Gewebemoleküle in kleinere Moleküle, d.h. in Abbauprodukte durch Spaltung kovalenter Bindungen überführt. Diese Moleküle können weiter untereinander reagieren und es entstehen neue Substanzen, die auf der Gewebeoberfläche vernetzen. Der Gesamtprozess führt letztendlich zu einer Stillung der Blutung.

Bei Patienten mit schlechten Blutgerinnungseigenschaften werden die thermischen und biochemischen Maßnahmen zur Blutstillung nicht zum gewünschten Ergebnis führen. Aus diesem Grund weist das Blutgerinnungsinstrument gemäß der vorliegenden Erfindung ein integriertes Zuführungs- und Dosierungssystem für Fibrinogen, einer Fibrinogenzubereitung oder einer anderen Substanz, die Einfluss auf das Blutgerinnungsverhalten nimmt, auf. Hierdurch ist gewährleistet, dass die Bildung eines Fibrinnetzwerks durch die Zuführung einer entsprechenden Hilfssubstanz unterstützt wird.

Das Blutstillungsinstrument gemäß der vorliegenden Erfindung umfasst vorzugsweise eine weitere Blutstillungskomponente in Form einer Erwärmungseinrichtung zur endogenen oder exogenen Erwärmung des Blutes während einer stillen elektrischen Entladung auf eine Temperatur unterhalb der Koagulationstemperatur von biologischem Gewebe, d.h. unterhalb von 60°C. Weiterhin ist vorzugsweise die thermische Blutstillungseinrichtung zur Kontakt-Koagulation oder zur Plasma-Koagulation, insbesondere zur Argon-Plasma-Koagulation ausgebildet. Besonders bevorzugt wird ein Blutstillungsinstrument, bei dem die Koagulationselektrode der thermischen Blutstillungseinrichtung gleichzeitig die Entladungselektrode der biochemischen Blutstillungseinrichtung bildet. Die Entladungselektrode der biochemischen Blutstillungseinrichtung kann dabei als Ringelektrode ausgebildet und koaxial zu der Koagulationselektrode der thermischen Blutstillungseinrichtung angeordnet sein. Weiterhin kann das Blutstillungsinstrument mindestens einen Gewebesensor aufweisen, der während des Einsatzes ein oder mehrerer Blutstillungskomponenten entstehende Gewebeeffekte erfasst. Der Gewebesensor kann beispielsweise in das chirurgische Instrument integriert sein. Denkbar ist es jedoch auch den Gewebesensor in das Chirurgiegerät zu integrieren oder den mindestens einen Gewebesensor als externe separate Einheit auszubilden. Weiterhin kann das Blutstillungsinstrument wenigstens einen Biosensor aufweisen, der Patienteninformationen erfasst, beispielsweise durch die Analyse einer Körperflüssigkeit des Patienten, so dass eine optimierte Einstellung und/oder Kombination der einzelnen Blutstillungskomponenten in Abhängigkeit von situations- und patientenbedingten Einflüssen erfolgt. Der wenigstens eine Biosensor ist vorzugsweise als Schwingquarzsensor bzw. als quartz crystal microbalance (QCM) Sensor ausgebildet, der aus einer Quarzscheibe mit beidseitig aufgedampften Goldelektroden besteht. Durch Anlegen einer elektrischen Wechselspannung an die beiden Goldelektroden entsteht innerhalb des Quarzes eine stehende akustische Transversalwelle. Dabei ist die Schwingungsfrequenz abhängig von der Massenanlagerung an der oberen Elektrode.

Zur Lösung der oben genannten Aufgabe wird auch eine Chirurgieeinrichtung zur Stillung von Blutungen mit den Merkmalen des Anspruchs 9 vorgeschlagen. Die Chirurgieeinrichtung dient insbesondere zur Stillung von Blutungen nach einer offenen, laparoskopischen oder endoskopischen Operation an einem Patienten und weist vorzugsweise folgende Komponenten auf:
- wenigstens einen Biosensor zur Erfassung von Bluteigenschaften eines Patienten während, vor oder zu Beginn eines operativen Eingriffs;
- ein Blutstillungsinstrument gemäß der oben genannten Beschreibung,
- wobei eine optimierte Einstellung und/oder Kombination der einzelnen Blutstillungskomponenten jeweils in Abhängigkeit von den durch den Biosensor erfassten Bluteigenschaften des Patienten erfolgt.

Durch die Chirurgieeinrichtung gemäß der vorliegenden Erfindung ergibt sich der Vorteil, dass die Stillung von Blutungen durch die Verwendung eines Biosensors jeweils in Abhängigkeit von den patienteneigenen Bluteigenschaften erfolgt, so dass eine Blutstillung noch effektiver erfolgen kann. Ansonsten ergeben sich darüber hinaus die Vorteile des Blutstillungsinstruments gemäß der vorliegenden Erfindung.

Die thermische Blutstillungskomponente ist vorzugsweise zur Gewebekoagulation durch Erzeugen einer Temperatur oberhalb der Koagulationstemperatur von biologischem Gewebe mittels einer Koagulationselektrode ausgebildet, wobei die Koagulationselektrode mit einem hochfrequenten Strom gespeist wird. Die thermische Blutstillungskomponente kann weiterhin zur Kontakt-Koagulation oder zur Plasma-Koagulation, insbesondere zur Argon-Plasma-Koagulation ausgebildet sein. Die biochemische Blutstillungskomponente ist hingegen vorzugsweise zur Erzeugung einer stillen elektrischen Entladung mittels wenigstens einer Entladungselektrode und einer Isoliereinrichtung ausgebildet, wobei die Isoliereinrichtung zwischen der Entladungselektrode und dem zu behandelnden Gewebe angeordnet ist und die Entladungselektrode mit einem HF-Strom gespeist wird. Darüber hinaus ist vorzugsweise eine weitere Blutstillungskomponente in Form einer Zufuhreinrichtung zum Zuführen von die Blutgerinnung beeinflussenden Stoffen an das zu behandelnde Gewebe vorgesehen. Eine weitere Blutstillungskomponente ist vorzugsweise in Form einer Zufuhreinrichtung zum Zuführen von Edelgas, insbesondere Argon, an das zu behandelnde Gewebe vorgesehen. Darüber hinaus kann noch eine weitere Blutstillungskomponente in Form einer Erwärmungseinrichtung zu endogenen oder exogenen Erwärmung des Blutes während einer stillen elektrischen Entladung auf eine Temperatur unterhalb der Koagulationstemperatur von biologischem Gewebe vorgesehen sein. Darüber hinaus kann die Chirurgieeinrichtung einen Gewebesensor aufweisen, der entstehende Gewebeeffekte während des Einsatzes ein oder mehrere Blutstillungskomponenten umfasst und der vorzugsweise im chirurgischen Instrument angeordnet ist. Eine Optimierung der Einstellung und/oder Kombination der einzelnen Blutstillungskomponenten erfolgt vorzugsweise auf der Grundlage der erfassten Werte des Gewebesensors und des Biosensors durch eine in der Chirurgieeinrichtung vorgesehene Steuereinheit "in Echtzeit".

Die Erfindung wird im Folgenden anhand der Zeichnung näher erläutert. Es zeigen:
- Fig. 1A: eine schematische Darstellung einer ersten Ausführungsform eines Blutstillungsinstruments zur aktiven Stillung von Blutungen gemäß der vorliegenden Erfindung in einem ersten Betriebszustand;
- Fig. 1B: eine schematische Darstellung des Blutstillungsinstruments gemäß Fig. 1A in einem zweiten Betriebszustand;
- Fig. 1C: eine schematische Darstellung des Blutstillungsinstruments gemäß Fig. 1A in einem dritten Betriebszustand;
- Fig. 2A: eine schematische Darstellung einer zweiten Ausführungsform eines Blutstillungsinstruments gemäß der vorliegenden Erfindung in einem ersten Betriebszustand;
- Fig. 2B: eine schematische Darstellung des Blutstillungsinstruments gemäß Fig. 2A in einem zweiten Betriebszustand;
- Fig. 2C: eine schematische Darstellung des Blutstillungsinstruments gemäß Fig. 2A in einem dritten Betriebszustand;
- Fig. 3A: eine schematische Darstellung einer weiteren Ausführungsform des Blutstillungsinstruments gemäß der vorliegenden Erfindung in einem ersten Betriebszustand;
- Fig. 3B: das Blutstillungsinstrument gemäß Fig. 3A in einem zweiten Betriebszustand;
- Fig. 3C: eine schematische Darstellung des Blutstillungsinstruments gemäß Fig. 3A in einem dritten Betriebszustand, und
- Fig. 4: eine schematische Darstellung einer Chirurgieeinrichtung gemäß der vorliegenden Erfindung.

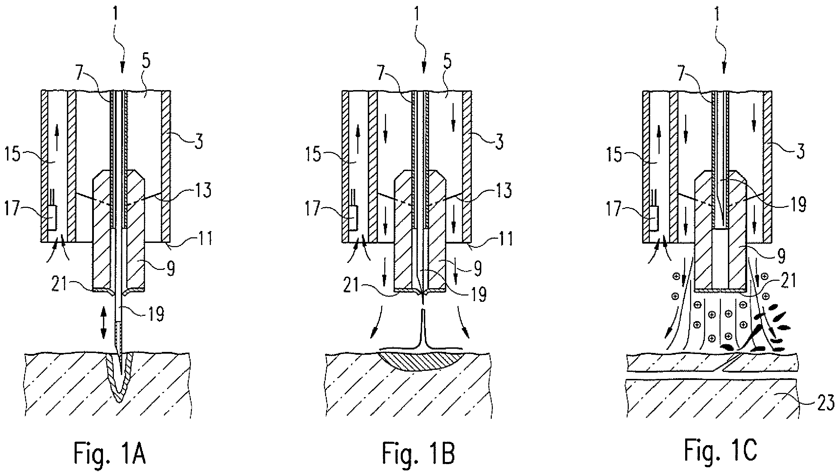

Fig. 1A zeigt eine schematische Darstellung einer ersten Ausführungsform des Blutstillungsinstruments 1 zur aktiven Stillung von Blutungen gemäß der vorliegenden Erfindung. Das Blutstillungsinstrument 1 umfasst ein Rohr oder einen Schlauch 3, der ein Lumen 5 einschließt. In dem Lumen ist ein Zufuhr- und Führungskanal 7 vorgesehen, der in ein distales Endstück 9 des Blutstillungsinstruments 1 hineinragt, wobei das distale Endstück 9 über das distale Ende 11 des Blutstillungsinstruments 1 hinausragt.

Sowohl der Zufuhr- und Führungskanal 7 als auch das distale Endstück 9 sind vorzugsweise zentrisch in dem Lumen 5 des Schlauchs 3 angeordnet. Das distale Endstück 9 ist mittels hier nur angedeuteter Verbindungselemente 13 mit dem Schlauch 3 verbunden. Parallel zu dem Lumen 5 und angrenzend an den Schlauch 3 ist ein Abzugskanal 15 vorgesehen, an dessen distalen Ende ein Gewebesensor 17 vorgesehen ist, der einen Gewebeeffekt, beispielsweise während der Operation entstehendes Rauchgas, erfassen kann. Das Emissionsgas wird durch den Abzugskanal 15 aus dem Operationsbereich entfernt.

In dem Zufuhr- und Führungskanal 7 ist eine Elektrode 19 vorgesehen, die in den Zufuhr- und Führungskanal 7 beweglich gelagert ist und eine am distalen Ende 21 des distalen Endstücks 9 befindliche Isoliereinrichtung 21 durchstoßen kann. Die Isoliereinrichtung 21 ist hierzu vorzugsweise elastisch ausgebildet und kehrt in seine Ausgangsposition zurück, sobald die Elektrode 19 vollständig in dem Zufuhr- und Führungskanal 7 angeordnet ist (s. Fig. 1C). Damit sich die Isoliereinrichtung 21 bei einer Krafteinwirkung der Elektrode 19 öffnet, weist sie vorzugsweise einen Schlitz oder dergleichen Öffnung auf.

Die Elektrode 19 ist in den vorliegenden Ausführungsbeispielen als Hohlnadel ausgebildet, mit der eine Blutprobe aus dem zu behandelnden Gewebe gewonnen wird, wie in Fig. 1A dargestellt ist. Mit der Hohlnadel kann auch ein Hochfrequenzschnitt am Gewebe durchgeführt werden. Die Elektrode 19 steht mit einem nicht dargestellten HF-Generator in Verbindung, der einen hochfrequenten Strom an die Elektrode liefert, sobald ein Hochfrequenzschnitt, eine Plasma-Koagulation oder eine andere elektrochirurgische Anwendung am Gewebe durchgeführt werden soll.

In Fig. 1B ist das Blutstillungsinstrument 1 in einem zweiten Betriebszustand dargestellt, bei dem die Spitze der Hohlnadel, d.h. der Elektrode 19 nur minimal durch die Isoliereinrichtung 21 über das distale Ende des distalen Endstücks 9 hinausragt, während der Elektrode 19 ein hochfrequenter Strom zugeführt wird. Gleichzeitig wird über das Lumen 5 bzw. über den Schlauch 3 ein Inertgas, insbesondere Argon, an das Gewebe geleitet, wodurch es zur Erzeugung eines Argon-Plasmas kommt. Sofern die Elektrode 19 also die Isoliereinrichtung 21 durchstößt und über das distale Ende des distalen Endstücks 9 in Richtung des Gewebes übersteht, wird eine thermische Blutstillungseinrichtung zur Gewebekoagulation durch Erzeugung einer Temperatur oberhalb der Koagulationstemperatur von biologischem Gewebe, d.h. oberhalb von 60°C mittels der Elektrode 19 aktiviert, wobei die Elektrode in diesem Betriebszustand als Koagulationselektrode wirkt. Sofern die an die Elektrode 19 zugeführte Energie gering ist, kann die thermische Blutstillungseinrichtung auch dazu dienen, ein Gas-Plasma zu erzeugen, welches das Gewebe lediglich schonend vorwärmt. Sie bildet dann gleichzeitig eine thermische Erwärmungseinrichtung.

Fig. 1C zeigt das Blutstillungsinstrument 1 in einem dritten Betriebszustand, in dem die Elektrode 19 vollständig in dem Zufuhr- und Führungskanal 7 angeordnet ist und somit die Isoliereinrichtung 21 den distalen Auslass des distalen Endstücks 9 vollständig verschließt, so dass die Elektrode 19 gegenüber dem Gewebe 23 isoliert ist. Die Isoliereinrichtung 21 ist in diesem Betriebszustand des Blutstillungsinstruments 1 zwischen der (Entladungs-)Elektrode 19 und dem zu behandelnden Gewebe angeordnet. Wird der Elektrode 19 in dieser Position ein HF-Strom zugeführt, wirkt sie als Entladungselektrode für die biochemische Blutstillung zur Erzeugung einer stillen elektrischen Entladung. Im Übrigen ist vorzugsweise auch das Endstück 9 isolierend ausgebildet. Denkbar ist es auch das Endstück einstückig mit der Isoliereinrichtung 21 auszubilden.

Während der in Fig. 1C gezeigten Blutungsstillung mittels der stillen elektrischen Entladung kann ein die Blutgerinnung beeinflussendes Fluid über das Lumen 5 oder über den Zufuhr- und Führungskanal 7 an das Gewebe geleitet werden.

In der in Fig. 1C gezeigten Position der Elektrode 19 wird folglich eine biochemische Blutstillungseinrichtung zur Erzeugung einer stillen elektrischen Entladung realisiert. Die Isoliereinrichtung 21 dient hierbei als Dielektrikum in einer Edelgasatmosphäre, wobei das Edelgas wiederum über das Lumen 5 in dem Zufuhrkanal 3 an das Gewebe 23 geleitet wird, um die benötigte Spannung zur Erzeugung der stillen elektrischen Entladung unterhalb von 4 kV zu halten.

Es zeigt sich somit, dass das Blutstillungsinstrument 1 gemäß der vorliegenden Ausführungsform sowohl eine thermische Blutstillungseinrichtung zur Gewebe-koagulation in Form der beweglich gelagerten Elektrode 19 aufweist und gleichzeitig eine biochemische Blutstillungseinrichtung zur Erzeugung einer stillen elektrischen Entladung mittels der Entladungselektrode 19, die hier gleichzeitig die Koagulationselektrode bildet sowie eine Isoliereinrichtung 21, wobei die Isoliereinrichtung zwischen der Entladungselektrode, hier also zwischen der Elektrode 19 und dem zu behandelnden Gewebe 23 angeordnet ist. Weiterhin ist eine Zufuhreinrichtung zum Zuführen von Edelgas und zum Zuführen von die Blutgerinnung beeinflussenden Stoffen an das zu behandelnde Gewebe in Form von dem Zufuhr- und Führungskanal 7 und das distale Endstück umgebende Lumen in dem Rohr 3 vorgesehen. Ein "Umschalten" zwischen den beiden Blutstillungseinrichtung erfolgt bei dem vorliegenden Ausführungsbeispiel durch eine Verlagerung der Elektrode 19 von einer ersten Position innerhalb des Zufuhr-und Führungskanals 7 in eine zweite Position außerhalb des Zufuhr- und Führungskanals 7.

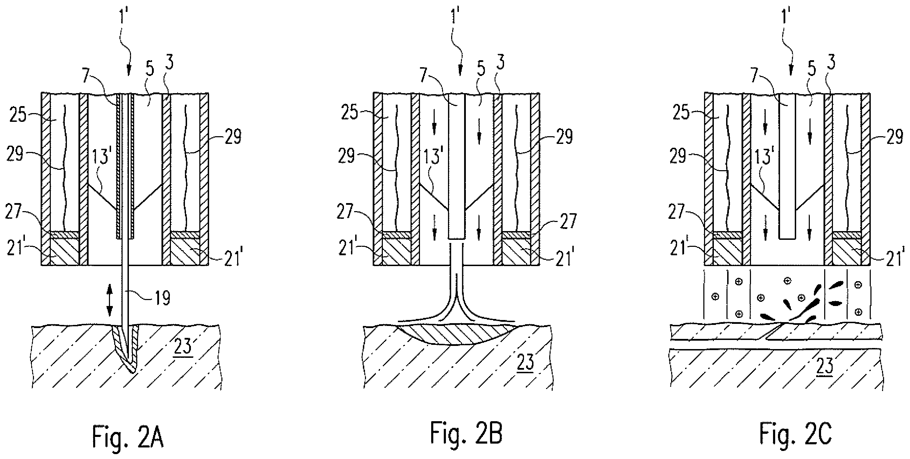

Eine weitere Ausführungsform eines Blutstillungsinstruments ist in den Fig. 2A bis 2C gezeigt. Das Blutstillungsinstrument 1' gemäß den Fig. 2A bis 2C unterscheidet sich von dem Blutstillungsinstrument 1 gemäß den Fig. 1A bis 1C insbesondere dadurch, dass kein distales Endstück in dem Blutstillungsinstrument 1' vorgesehen ist, sondern vielmehr der Zufuhr- und Führungskanal 7 mit der darin gelagerten Elektrode 19, die wiederum als Hohlnadel insbesondere zur Entnahme einer Blutprobe ausgebildet ist, mittels Verbindungselementen 13' an dem Schlauch 3 befestigt ist. Auch bei dieser Ausführungsform der Erfindung ist die Elektrode 19 gleichzeitig als Hohlnadel ausgebildet und beweglich in dem Führungskanal 7 gelagert.

Darüber hinaus unterscheidet sich das Blutstillungsinstrument 1' gemäß den Fig. 2A bis 2C von der Ausführungsform in den Fig. 1A bis 1C dadurch, dass die Isoliereinrichtung 21' als ringförmiger Körper ausgebildet ist, der am distalen Ende eines das Lumen 5 bzw. das Rohr 3 koaxial umgebenden Ringraums bzw. Kanals 25 angeordnet ist. Am proximalen Ende der Isoliereinrichtung 21' ist eine ringförmige Entladungselektrode 27 vorgesehen, die flächig an der Isoliereinrichtung 21 anliegt und folglich ebenfalls koaxial den Schlauch 3 in dem Kanal 25 umgibt. Die Entladungselektrode 27 ist über Zuleitungen 29 mit einer entsprechenden Spannungs- bzw. Stromquelle, insbesondere mit dem HF-Generator eines Chirurgiegeräts verbunden.

Insgesamt zeigt sich, dass bei der vorliegenden Ausführungsform die Koagulationselektrode, d.h. die Elektrode 19 und die Entladungselektrode 27 der biochemischen Blutstillungseinrichtung als getrennte Elemente ausgebildet sind.

Fig. 2B zeigt das Blutstillungsinstrument 1' in einem Betriebszustand, in dem die thermische Blutstillungseinrichtung zur Gewebekoagulation durch Erzeugung einer Temperatur oberhalb der Koagulationstemperatur von biologischem Gewebe mittels der Koagulationselektrode 19 stattfinden kann. In diesem Fall ist die als Elektrode 19 fungierende Hohlnadel wiederum in den Zufuhr- und Führungskanal 7 eingezogen, während die Elektrode 19 (in Fig. 2B nicht sichtbar) mit einem hochfrequenten Strom beaufschlagt wird. Gleichzeitig wird durch das Lumen 5 ein Inertgas, insbesondere Argon, an das zu behandelnde Gewebe 23 geführt, so dass ein Argon-Plasma zwischen dem Blutstillungsinstrument 1' und dem Gewebe 23 entsteht.

Fig. 2C zeigt das Blutstillungsinstrument 1 gemäß der vorliegenden Ausführungsform in einem aktivierten Betriebszustand der biochemischen Blutstillungseinrichtung. In diesem Betriebszustand wird durch das Lumen 5 wiederum ein Inertgas, insbesondere ein Edelgas in Form von Argon an das Gewebe 23 herangeführt, während gleichzeitig die Entladungselektrode 27 über die Zuleitung 29 mit einem HF-Strom beaufschlagt wird, so dass sich eine stille elektrische Entladung, d.h. ein kaltes Plasma zwischen der Entladungselektrode 27 und dem als zweite Elektrode fungierenden Gewebe 23 ausbildet. Über das Lumen 5 des Schlauchs 3 kann gleichzeitig als Zufuhreinrichtung zum Zuführen von die Blutgerinnung beeinflussenden Stoffen an das zu behandelnde Gewebe ausgebildet sein.

Das Blutstillungsinstrument 1' gemäß den Fig. 2A bis 2C ermöglicht ein Hochfrequenzschneiden von biologischem Gewebe mit zentrisch angeordneter ausfahrbarer HF-Elektrode 19, die insbesondere die Form einer Hohlnadel aufweist. Insgesamt kann durch das Blutstillungsinstrument 1' sowohl eine thermische Blutstillung mittels der Koagulationselektrode 19 als auch eine biochemische Blutstillung mittels der Entladungselektrode 27 realisiert werden. Das Blutstillungsinstruments 1' gemäß der vorliegenden Erfindung ist darüber hinaus zum Zuführen von Flüssigkeiten wie z.B. Addukten geeignet, welche die nicht-thermische, d.h. die biochemische Blutstillung unterstützen sollen, insbesondere zum Zuführung von Fibrinogen, Thrombin, Gerinnungsfaktoren oder Ähnlichem. Darüber hinaus ist das Instrument zum Zuführen eines Fluids zum Freispülen des OP-Situs nach einer Blutung geeignet.

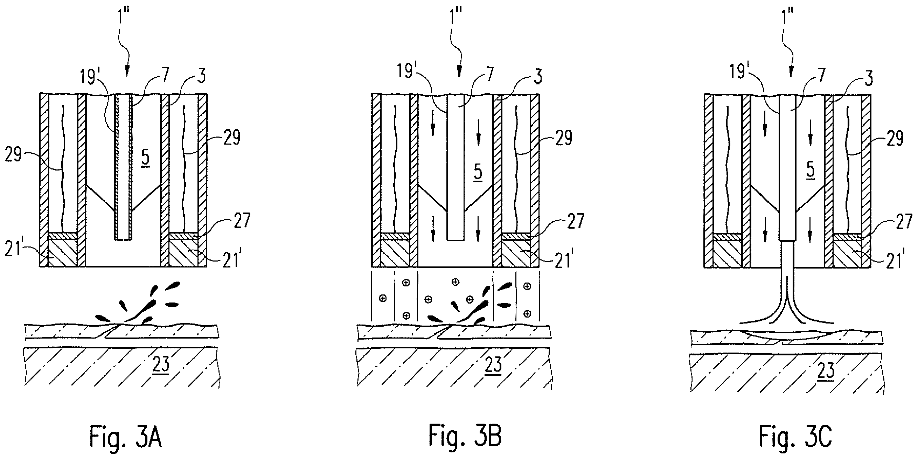

Die Fig. 3A bis 3C zeigen eine weitere Ausführungsform eines Blutstillungsinstruments gemäß der Erfindung. Im Gegensatz zu dem Blutstillungsinstrument 1' gemäß den Fig. 2A bis 2C ist der Zufuhr- und Führungskanal 7 vorliegend als Elektrode 19' ausgebildet, umfasst also keine darin gelagerte Hohlnadel. Der Zufuhr- und Führungskanal 7 kann bei dieser Ausführungsform gleichzeitig als Zuleitung für eine Flüssigkeit, insbesondere unter Erzeugung eines Kegelstrahls dienen, durch die insbesondere die Zuführung von die Blutgerinnung beeinflussenden Stoffen an das zu behandelnde Gewebe 23 gewährleistet ist. Ansonsten entspricht die Ausführungsform und ihre Funktionsweise der Ausführungsform nach den Fig. 2A bis 2C, auf die hiermit verwiesen wird.

Aus Fig. 3B ist noch erkennbar, dass bei einer stillen elektrischen Entladung, d.h. bei einer aktiven biochemischen Blutstillungseinrichtung die Zufuhreinrichtung zum Zuführen von die Blutgerinnung beeinflussenden Stoffen an das zu behandelnde Gewebe 23 über den Zufuhrkanal 7 aktiviert sein kann. Eine Zufuhr von Edelgas kann wiederum über das Lumen 5 erfolgen. Es versteht sich, dass die Zufuhreinrichtung zum Zuführen von die Blutgerinnung beeinflussenden Stoffen auch unabhängig vom jeweiligen Blutungsstillungsmodus aktiviert sein kann.

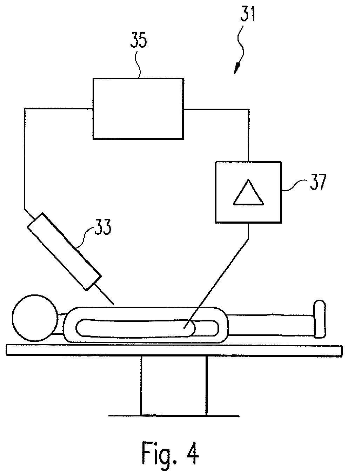

Fig. 4 zeigt noch eine schematische Darstellung einer Chirurgieeinrichtung 31 gemäß der vorliegenden Erfindung. Die Chirurgieeinrichtung 31 umfasst ein chirurgisches Instrument, insbesondere ein elektrochirurgisches Instrument 33, eine Steuereinrichtung 35 sowie einen Biosensor 37. Der Biosensor 37 ist dazu ausgelegt, die Bluteigenschaften eines Patienten während, vor oder zu Beginn eines operativen Eingriffs zu erfassen. Hierzu umfasst der Biosensor vorzugsweise ein piezoelektrisches Element oder dergleichen, um festzustellen, welche Blutgerinnungseigenschaften ein Patient aufweist. Die erfassten Daten des Biosensors können dann an eine Steuereinheit 35 weitergeleitet werden, die entsprechende Einstellungen des HF-Generators bzw. anderen operativen Hilfsmitteln zur Durchführung des operativen Eingriffs vornimmt.

Die erfassten Daten des Biosensors werden vorzugsweise dazu herangezogen, eine entsprechende Einstellung und insbesondere eine entsprechende Kombination der thermischen Blutstillungskomponente und der biochemischen Blutstillungskomponente sowie der anderen Blutstillungskomponenten vorzunehmen. Durch den Biosensor kann beispielsweise das Blutbild oder die Blutgerinnungseigenschaften des Patienten analysiert werden. Beispielsweise kann ein "quartz crystal microbalance"-Sensor (Gehring et al.) verwendet werden, um die Prothrombin-Zeit (PT-Zeit) zu ermitteln, welche eine Aussage über die patientenindividuelle Blutgerinnungseigenschaft ermöglicht. Auf diese Weise kann eine optimale Einstellung der Chirurgieeinrichtung aus biochemischer und thermischer Blutstillung sowie die Zuführung von Addukten, also die Blutgerinnung beeinflussenden Mitteln, sowie die Zufuhr eines Edelgases ermittelt und von der Chirurgieeinrichtung bereitgestellt werden. Dies bedeutet, dass die einzelnen Komponenten je nach Gerinnungssignal des Biosensors in unterschiedlicher Gewichtung gleichzeitig oder hintereinander angewandt werden können, wobei nicht jede Komponente bei jedem operativen Eingriff zwingend verwendet werden muss.

Mittels des Gewebesensors 17 wird ein tatsächlich entstehender Gewebeeffekt während einer Operation detektiert. Dies kann beispielsweise über die qualitative oder quantitative Erfassung des während der Anwendung entstehenden Rauchgases oder der Detektion der elektromagnetischen Strahlung einer Anwendung einer medizinischen Plasmaanwendung geschehen. Über die Steuereinheit kann aus dem biochemischen Sensorsignal und dem Gewebeeffektsignal eine Echtzeitoptimierung der chirurgischen Anwendung erfolgen. Letztendlich ergibt sich daraus eine patientenspezifische Möglichkeit, Blutstillungen bei minimaler Schädigung des umliegenden Gewebes durchzuführen.

### Bezugszeichenliste

- 1: Blutstillungsinstrument
- 1': Blutstillungsinstrument
- 1": Blutstillungsinstrument
- 3: Schlauch
- 5: Lumen
- 7: Zufuhr- und Führungskanal
- 9: Distales Endstück
- 11: Distales Ende
- 13: Verbindungselement
- 13': Verbindungselement
- 15: Abzugskanal
- 17: Gewebesensor
- 19: Elektrode
- 19': Elektrode
- 21: Isoliereinrichtung
- 21': Isoliereinrichtung
- 23: Gewebe
- 25: Kanal
- 27: Entladungselektrode
- 29: Zuleitung
- 31: Chirurgieeinrichtung
- 33: Chirurgisches Instrument
- 35: Steuereinheit
- 37: Biosensor

## Patentansprüche

1. Blutstillungsinstrument (1, 1', 1") zur aktiven Stillung von Blutungen, insbesondere nach einer offenen, laparoskopischen oder endoskopischen Operation an einem Patienten, aufweisend mehrere Blutstillungskomponenten, wobei wenigstens die folgenden Blutstillungskomponenten vorgesehen sind:
a. eine thermische Blutstillungseinrichtung zur Gewebekoagulation durch Erzeugen einer Temperatur oberhalb der Koagulations-temperatur von biologischem Gewebe mittels einer Koagulationselektrode (19);
b. eine biochemische Blutstillungseinrichtung zur Erzeugung einer stillen elektrischen Entladung mittels wenigstens einer Entladungselektrode (19, 27) und einer Isoliereinrichtung (21, 21'), wobei die Isoliereinrichtung zwischen der Entladungselektrode und dem zu behandelnden Gewebe (23) angeordnet ist;
c. ein integriertes Zuführungs- und Dosierungssystem für Fibrinogen oder eine Fibrinogenzubereitung, und
d. eine Zufuhreinrichtung (5) zum Zuführen von Edelgas an das zu behandelnde Gewebe.

2. Blutstillungsinstrument nach Anspruch 1,
**dadurch gekennzeichnet, dass**
das Blutstillungsinstrument eine weitere Blutstillungskomponente in Form einer Erwärmungseinrichtung zur endogenen oder exogenen Erwärmung des Blutes während einer stillen elektrischen Entladung auf eine Temperatur unterhalb der Koagulationstemperatur von biologischem Gewebe aufweist.

3. Blutstillungsinstrument nach Anspruch 1 oder 2,
**dadurch gekennzeichnet, dass**
die thermische Blutstillungseinrichtung zur Kontaktkoagulation oder zur Plasma-Koagulation, insbesondere zur Argon-Plasma-Koagulation ausgebildet ist.

4. Blutstillungsinstrument nach einem der vorhergehenden Ansprüche,
**dadurch gekennzeichnet, dass**
die Koagulationselektrode (19) der thermischen Blutstillungseinrichtung gleichzeitig die Entladungselektrode der biochemischen Blutstillungseinrichtung bildet.

5. Blutstillungsinstrument nach einem der Ansprüche 1 bis 3,
**dadurch gekennzeichnet, dass**
die Entladungselektrode (27) der biochemischen Blutstillungseinrichtung als Ringelektrode ausgebildet und koaxial zu der Koagulationselektrode (19) der thermischen Blutstillungseinrichtung angeordnet ist.

6. Blutstillungsinstrument nach einem der vorhergehenden Ansprüche,
**dadurch gekennzeichnet, dass**
es mindestens einen Gewebesensor (17) aufweist, der während des Einsatzes ein oder mehrerer Blutstillungskomponenten entstehende Gewebeeffekte erfasst.

7. Blutstillungsinstrument nach einem der vorhergehenden Ansprüche,
**dadurch gekennzeichnet, dass**
eine optimale Einstellung und/oder Kombination der einzelnen Blutstillungskomponenten in Abhängigkeit von situations- und patientenbedingten Einflüssen erfolgt.

8. Blutstillungsinstrument nach Anspruch 7,
**dadurch gekennzeichnet, dass**
die patientenbedingten Einflüsse in Form von durch wenigstens einen Biosensor (37) erfasste Patienteninformationen vorliegen.

9. Chirurgieeinrichtung zur Stillung von Blutungen, insbesondere nach einer offenen, laparoskopischen oder endoskopischen Operation an einem Patienten, aufweisend:
a. wenigstens einen Biosensor zur Erfassung von Bluteigenschaften eines Patienten während, vor oder zu Beginn eines operativen Eingriffs;
b. ein Blutstillungsinstrument nach einem der vorhergehenden Ansprüche, und
c. wobei die Chirurgieeinrichtung derart ausgebildet ist, dass eine optimale Einstellung und/oder Kombination der einzelnen Blutstillungskomponenten jeweils in Abhängigkeit von den durch den Biosensor erfassten Bluteigenschaften des Patienten erfolgt.

10. Chirurgieeinrichtung nach Anspruch 9,
**dadurch gekennzeichnet, dass** die Koagulationselektrode mit einem HF-Strom gespeist wird.

11. Chirurgieeinrichtung nach Anspruch 9 bis 10, **dadurch gekennzeichnet, dass** die Entladungselektrode mit einem HF-Strom gespeist wird.

12. Chirurgieeinrichtung nach den Ansprüchen 9 und 6, **dadurch gekennzeichnet, dass** die Chirurgieeinrichtung derart ausgebildet ist, dass eine Optimierung der Einstellung und/oder Kombination der einzelnen Blutstillungskomponenten auf der Grundlage der erfassten Werte des Gewebesensors und des Biosensors durch eine in der Chirurgieeinrichtung vorgesehene Steuereinheit in "Echtzeit" erfolgt.

## Claims

1. Hemostasis instrument (1, 1', 1") for actively stopping bleeding, in particular after an open, laparoscopic or endoscopic operation on a patient, comprising a plurality of hemostasis components, wherein at least the following hemostasis components are provided:
a. a thermal hemostasis apparatus for tissue coagulation by generating a temperature above the coagulation temperature of biological tissue by means of a coagulation electrode (19);
b. a biochemical hemostasis apparatus for generating a dielectric barrier discharge by means of at least one discharge electrode (19, 27) and an insulation apparatus (21, 21'), wherein the insulation apparatus is arranged between the discharge electrode and the tissue (23) to be treated;
c. an integrated supply and metering system for fibrinogen or a fibrinogen preparation, and
d. a supply apparatus (5) for supplying noble gas to the tissue to be treated.

2. Hemostasis instrument according to Claim 1, **characterized in that** the hemostasis instrument comprises a further hemostasis component in the form of a heating apparatus for endogenous or exogenous heating of the blood during a dielectric barrier discharge to a temperature below the coagulation temperature of biological tissue.

3. Hemostasis instrument according to Claim 1 or 2, **characterized in that** the thermal hemostasis apparatus is designed for contact coagulation or for plasma coagulation, in particular for argon plasma coagulation.

4. Hemostasis instrument according to one of the preceding claims, **characterized in that** the coagulation electrode (19) of the thermal hemostasis apparatus at the same time forms the discharge electrode of the biochemical hemostasis apparatus.

5. Hemostasis instrument according to one of Claims 1 to 3, **characterized in that** the discharge electrode (27) of the biochemical hemostasis apparatus is designed as a ring electrode and arranged coaxially with respect to the coagulation electrode (19) of the thermal hemostasis apparatus.

6. Hemostasis instrument according to one of the preceding claims, **characterized in that** it comprises at least one tissue sensor (17) which captures tissue effects generated during the use of one or more hemostasis components.

7. Hemostasis instrument according to one of the preceding claims, **characterized in that** there is an optimum setting and/or combination of the individual hemostasis components, depending on situation- and patient-dependent influences.

8. Hemostasis instrument according to Claim 7, **characterized in that** the patient-dependent influences are available in the form of patient information captured by at least one biosensor (37).

9. Surgical apparatus for stopping bleeding, in particular after an open, laparoscopic or endoscopic operation on a patient, comprising:
a. at least one biosensor for capturing blood properties of a patient during, before or at the start of a surgical intervention;
b. a hemostasis instrument according to one of the preceding claims, and
c. wherein the surgical apparatus is configured such that there is an optimum setting and/or combination of the individual hemostasis components, respectively depending on the blood properties of the patient captured by the biosensor.

10. Surgical apparatus according to Claim 9, **characterized in that** the coagulation electrode is fed with RF-current.

11. Surgical apparatus according to Claim 9 to 10, **characterized in that** the discharge electrode is fed with RF-current.

12. Surgical apparatus according to Claims 9 and 6, **characterized in that** the surgical apparatus is configured such that an optimization of the setting and/or combination of the individual hemostasis components is brought about on the basis of the captured values of the tissue sensor and of the biosensor in "real-time" by a control unit provided in the surgical apparatus.

## Revendications

1. Instrument hémostatique (1, 1', 1") permettant de stopper activement des saignements, en particulier après une intervention laparoscopique ou endoscopique ouverte chez un patient, et comportant plusieurs composants hémostatiques, dans lequel il est au moins prévu les composants hémostatiques suivants :
a. un dispositif hémostatique thermique pour la coagulation tissulaire en générant une température supérieure à la température de coagulation du tissu biologique au moyen d'une électrode de coagulation (19) ;
b. un dispositif hémostatique biochimique destiné à générer une décharge électrique hémostatique au moyen d'au moins une électrode de décharge (19, 27) et d'un dispositif isolant (21, 21'), dans lequel le dispositif isolant est disposé entre l'électrode de décharge et le tissu (23) à traiter ;
c. un système intégré d'amenée et de dosage de fibrinogène ou d'une préparation de fibrinogène ; et
d. un dispositif d'amenée (5) servant à amener un gaz inerte sur le tissu à traiter.

2. Instrument hémostatique selon la revendication 1,
**caractérisé en ce que** l'instrument hémostatique comprend un autre composant hémostatique sous la forme d'un dispositif de chauffage destiné au chauffage endogène ou exogène du sang pendant une décharge électrique hémostatique à une température inférieure à la température de coagulation du tissu biologique.

3. Instrument hémostatique selon la revendication 1 ou 2,
**caractérisé en ce que** le dispositif hémostatique thermique est conçu pour la coagulation par contact ou la coagulation plasmatique, en particulier pour la coagulation argon-plasma.

4. Instrument hémostatique selon l'une des revendications précédentes,
**caractérisé en ce que** l'électrode de coagulation (19) du dispositif hémostatique thermique forme simultanément l'électrode de décharge du dispositif hémostatique biochimique.

5. Instrument hémostatique selon l'une des revendications 1 à 3,
**caractérisé en ce que** l'électrode de décharge (27) du dispositif hémostatique biochimique est réalisée sous la forme d'une électrode annulaire et est disposée coaxialement par rapport à l'électrode de coagulation (19) du dispositif hémostase thermique.

6. Instrument hémostatique selon l'une des revendications précédentes,
**caractérisé en ce qu'**il comporte au moins un capteur tissulaire (17) qui détecte les effets tissulaires survenant pendant l'utilisation d'un ou de plusieurs composants hémostatiques.

7. Instrument hémostatique selon l'une des revendications précédentes,
**caractérisé en ce qu'**un ajustement et/ou une combinaison optimale des différents composants hémostatiques est effectué en fonction d'influences liées à la situation et au patient.

8. Instrument hémostatique selon la revendication 7,
**caractérisé en ce que** les influences liées au patient sont présentes sous la forme d'informations concernant le patient détectées par au moins un biocapteur (37).

9. Dispositif chirurgical permettant de stopper des saignements, en particulier après une intervention chirurgicale laparoscopique ou endoscopique ouverte chez un patient, comportant :
a. au moins un biocapteur destiné à détecter des caractéristiques sanguines d'un patient pendant, avant ou au début d'une intervention ;
b. un instrument hémostatique selon l'une des revendications précédentes ; et
c. dans lequel le dispositif chirurgical est conçu de telle sorte qu'un ajustement et/ou une combinaison optimale des différents composants hémostatiques soit respectivement effectuée en fonction des propriétés hémostatiques du patient, détectées par le biocapteur.

10. Dispositif chirurgical selon la revendication 9, **caractérisé en ce que** l'électrode de coagulation est alimentée en courant HF.

11. Dispositif chirurgical selon les revendications 9 à 10, **caractérisé en ce que** l'électrode de décharge est alimentée en courant HF.

12. Dispositif chirurgical selon les revendications 9 et 6,
**caractérisé en ce que** le dispositif chirurgical est conçu de telle sorte que l'ajustement et/ou la combinaison des différents composants hémostatiques soit optimisé en "temps réel" sur la base des valeurs détectées par le capteur tissulaire et le biocapteur, par une unité de commande prévue dans le dispositif chirurgical.
